# EUROPEAN PATENT SPECIFICATION

(11) **EP 2 695 610 B1**
(45) Date of publication and mention of the grant of the patent: **06.03.2019**
(21) Application number: 13177359.0
(22) Date of filing: 22.07.2013
(51) Int. Cl.: A61K 38/39, A23L 27/12, A23L 33/105, A23L 33/15, A23L 33/16, A23L 33/17

(54) **Food supplement for improving connective tissue health**
Nahrungsergänzung zur Verbesserung der Bindegewebegesundheit
Complément alimentaire pour améliorer la santé d'un tissu conjonctif

(30) Priority: 08.08.2012 IT TO20120714
(43) Date of publication of application: 12.02.2014
(73) Proprietor: Minerva Research Labs Ltd, London W1J 7BU (GB)
(72) Inventor: SANGUINETTI, Catone Tony, London, W1J 7BU (GB); AUNG, Thein, London, W1J 7BU (GB)
(74) Representative: Freyria Fava, Cristina

(56) References cited:
- WO-A1-2005/074719
- US-A1- 2003 069 171
- US-A1- 2009 269 424
- DATABASE WPI Week 200904 Thomson Scientific, London, GB; AN 2009-A87362 XP002687524, & KR 100 846 125 B1 (BIO SPECTRUM INC) 15 July 2008 (2008-07-15)
- DATABASE WPI Week 200872 Thomson Scientific, London, GB; AN 2008-M23671 XP002687605, & JP 2008 237070 A (ISSEI KAMABOKO KK) 9 October 2008 (2008-10-09)
- MORI ET AL: "Inhibition of elastase activity by essential oils in vitro", JOURNAL OF COSMETIC DERMATOLOGY, vol. 1, 2003, pages 183-187,

## Description

### Field of the invention

This disclosure concerns a food supplement particularly suitable for improving the health of the connective tissue.

### Background of the invention

The main functions of the skin are to provide to the body a mechanical protection and a chemical barrier able to restrain the foreign substance penetration, to prevent water or endogenous fluid loss and to keep a constant temperature. In addition, the skin also protect against ultraviolet irradiation and pathogen invasion.

Skin is subject to deterioration through dermatological disorders, environmental condition (wind, air conditioning, heating) or through the normal ageing process which may be accelerated by exposure of the skin to sun (photoageing).

In recent years the demand for cosmetic compositions and cosmetic methods for improving the appearance and condition of the skin has grown enormously.

The cosmetic and dermatological industry aims to prevent the skin dehydration, the appearance of dry skin or of fine lines and wrinkles by topical application of creams and lotions containing ingredients able to improve the skin quality.

This effect is, however, only local and the benefit is only provided to the part of the skin where the lotion or the cream are applied, that is on the outer skin layer, namely the epidermis.

In contrast, the dermis - the layer localized between the epidermis and the subcutaneous tissues - which consist of connective tissue and confers elasticity and firmness to the skin is not easily achievable by local application.

The use of ingredients that can improve the skin condition is beneficial especially when they are orally administrated thanks to the blood and interstitial fluids delivery system that distribute them to the whole body and to the entire thickness of the skin.

### Summary of the invention

The object of this disclosure is to provide a food supplement for improving connective tissue health, particularly by increasing connective tissue cells proliferation and synthesis of extracellular matrix (ECM) proteins through the synergistic effects exerted by its ingredients.

The present disclosure provides a food supplement comprising collagen, at least one glucosamine derivative selected from glucosamine hydrochloride and N-acetylglucosamine, L-carnitine, hyaluronic acid, a black pepper extract and a maca extract. The food supplement herein described may also include vitamins, minerals, additives, and flavouring substances.

### Detailed description of the invention

The present disclosure provides a food supplement comprising collagen, at least one glucosamine derivative selected from glucosamine hydrochloride and N-acetylglucosamine, L-carnitine, hyaluronic acid, a black pepper extract and a maca extract.

The food supplement herein described is able to stimulate connective tissue cells. In addition, it also exerts an anti-ageing effect.

The food supplement herein described can be realized both in liquid or solid form, i.e. by mixing the ingredients in solid form or by lyophilizing the liquid preparation.

The food supplement herein described comprises collagen in an amount between 1 and 10 g/50 ml, preferably in an amount between 2 and 7.5 g/50 ml, more preferably in an amount between 3 and 5 g/50 ml. The collagen used in the liquid food supplement can be hydrolyzed collagen, preferably deriving from a fish source.

The hyaluronic acid is present in an amount between 0.25 and 10 mg/50 ml, preferably in an amount between 0.75 and 6 mg/50 ml, more preferably in an amount between 1 and 3 mg/50 ml.

The glucosamine derivatives include glucosamine hydrochloride and N-acetylglucosamine.

Glucosamine hydrochloride is present in an amount between 1 and 3000 mg/50 ml, preferably in an amount between 500 and 1800 mg/50 ml, more preferably between 750 and 1250 mg/50 ml.

N-acetylglucosamine is present in an amount between 1 and 30 mg/50 ml, preferably in an amount between 2 and 15 mg/50 ml, more preferably in an amount between 3 and 7 mg/50 ml.

L-carnitine is present in an amount between 1 and 400 mg/50 ml, preferably between 100 and 300 mg/50 ml, more preferably between 150 and 250 mg/50 ml.

Collagen and hyaluronic acid are vital components naturally present in healthy skin tissues.

Collagen is the main structural protein of the skin and confers tensile strength and elasticity.

Hyaluronic acid is able to retain and hold moisture playing an important role in maintaining hydration and a youthful look of the skin.

It has been shown that both collagen and hyaluronic acid decline with age leading to a loss of the skin elasticity and to the formation of fine lines and wrinkles.
Glucosamine hydrochloride and N-acetylglucosamine are a source of glucosamine, which is an essential component of the connective tissue. Glucosamine provides the building blocks for the formation of hyaluronic acid, which is beneficial to skin and joint hydration and is essential to provide cartilage with its deformable resilience.

L-carnitine is mainly found in the skeletal muscle tissue and plays a key role in muscle bioenergetic. L-carnitine has been shown to have a potential role in tissue recovery after intense regular physical exercise

Due to an unexpected synergy among the effects exerted by its main ingredients, i.e. the collagen, the glucosamine derivatives, the L-carnitine and the hyaluronic acid, the food supplement herein described leads to an improvement of connective tissue structure and hydration.

The black pepper extract, known to enhance bioavailability of several substances by increasing their gastrointestinal absorption, can be present in an amount between 0.2 and 5 mg/50 ml, preferably between 0.8 and 4 mg/50 ml.

The maca extract can be present in an amount of 1 to 40 mg/50 ml, preferably 15 to 25 mg/50 ml.

The present Inventors found that adding the black pepper extract and the maca extract to the food supplement a further potentiating effect was obtained: 1) increased bioavailability of active ingredients; 2) improved physical and mental conditions.

In a further embodiment of the present disclosure the liquid food supplement herein described also includes at least one among: vitamins, minerals, additives and flavouring substances.

The vitamins useful in the food supplement are any vitamins known to have a health benefit to consumers.

Preferably, the vitamin is selected from the group consisting of vitamin B₁, vitamin B₂, vitamin B₃, vitamin B₆, vitamin B₁₂, vitamin C, vitamin D, biotin and other water soluble vitamins.

Vitamin B₆ brings energy and vitality. Vitamin B₃ can contribute to the reduction of tiredness and fatigue. Vitamin C contributes to normal collagen formation and the normal function of bones, teeth, cartilage, gums, skin and blood vessels. Vitamin D contributes to the maintenance of muscle function. Biotin contributes to the maintenance of hair.

The minerals selected for the liquid food supplement are known for their beneficial effects to hair.

Preferably minerals are chosen between Zinc and Copper. Zinc contributes to the maintenance of hair. Copper contributes to the maintenance of hair and skin pigmentation. Copper contributes to the maintenance of connective tissue.

The additive is preferably selected among citric acid anhydrous, phosphoric acid, lactic acid, tartaric acid, DL-malic acid and sucralose.

Flavouring substances may be selected among apple essential oil and mango essential oil.

Lactones, esters, aliphatic higher alcohols, ketones, aromatic aldehydes, aromatic alcohols, thioethers, fatty acids, propylene glycol, ethanol, glycerol (glycerine) were used to formulate the flavor base.

Table 1 reports the content range of the ingredients that can be included in the water based food supplement as described above.

**Table 1**

| **Ingredients** | **Content range/50 ml** |
|---|---|
| Collagen (hydrolysed fish collagen) | 1-10 g |
| Hyaluronic acid | 0.25-10 mg |
| Glucosamine hydrochloride | 1-3000 mg |
| N-acetylglucosamine | 1-30 mg |
| L-carnitine, | 1-400 mg |
| Maca extract | 1-40 mg |
| Black pepper extract | 0.2-5 mg |
| Vitamins | 0-800 mg |
| Additives | 0-1100 mg |
| Minerals | 0-20 mg |
| Flavouring substances | 0-400 mg |

### Example 1. (not part of the invention)

The food supplement in liquid form comprising the ingredients reported in Table 2 below has been prepared first by mixing and dissolving at a temperature of 60±5°C in a mixing tank water, hyaluronic acid, hydrolysed collagen, glucosamine hydrochloride, N-acetylglucosamine, L-carnitine, citric acid anhydrous, DL-malic acid, sucralose, pyridoxine hydrochloride (vitamin B₆) ascorbic acid (vitamin C), vitamin B₃ (niacin), vitamin D, vitamin B₁₂ (hydroxocobalamin), D-Biotin, Zinc and Copper. Then water is added into the mixing tank lowering the temperature to 50±5°C. Finally, the flavouring substances are also included. After mixing all the ingredients, water is added in order to adjust the volume to 50 ml.

**Table 2**

| **Ingredients** | **Content/50 ml** |
|---|---|
| Collagen (hydrolysed fish collagen) | 4 g |
| Glucosamine hydrochloride | 1 g |
| N-acetylglucosamine | 5 mg |
| L-carnitine, | 200 mg |
| Hyaluronic acid | 5 mg |
| Vitamin B₆ (Pyridoxine Hydrochloride) | 1.7 mg |
| Vitamin B₃ | 2.4 mg |
| Vitamin C (Ascorbic acid) | 150 mg |
| Biotin | 0.05 mg |
| Vitamin D | 0.005 mg |
| Vitamin B₁₂ | 0.00038 mg |
| Zinc | 1.5 mg |
| Copper | 0.15 mg |
| DL-Malic acid | 100 mg |
| Sucralose | 5 mg |
| Citric acid anhydrous | 675 mg |
| Flavouring substances | 140 mg |
| Water | 92 g |

### Example 2.

The food supplement in liquid form comprising the ingredients reported in Table 3 below has been prepared first by mixing and dissolving at a temperature of 60±5°C in a mixing tank water, hyaluronic acid, hydrolysed collagen, glucosamine hydrochloride, N-acetylglucosamine, L-carnitine, citric acid anhydrous, DL-malic acid, black pepper extract, sucralose, pyridoxine hydrochloride (vitamin B₆), ascorbic acid (vitamin C), maca, vitamin B₃ (niacin), vitamin D, vitamin B₁₂ (hydroxocobalamin), D-Biotin, Zinc and Copper. Then water is added into the mixing tank lowering the temperature to 50±5°C. Finally, the flavouring substances are also included. After mixing all the ingredients, water is added in order to adjust the volume to 50 ml.

**Table 3**

| **Ingredients** | **Content/50 ml** |
|---|---|
| Collagen (hydrolysed fish collagen) | 4 g |
| Glucosamine hydrochloride | 1 g |
| N-acetylglucosamine | 5 mg |
| L-carnitine, | 200 mg |
| Hyaluronic acid | 5 mg |
| Maca extract | 20 mg |
| Vitamin B₆ (Pyridoxine Hydrochloride) | 1.7 mg |
| Vitamin B₃ | 2.4 mg |
| Vitamin C (Ascorbic acid) | 150 mg |
| Biotin | 0.05 mg |
| Vitamin D | 0.005 mg |
| Vitamin B₁₂ | 0.00038 mg |
| Zinc | 1.5 mg |
| Copper | 0.15 mg |
| DL-Malic acid | 100 mg |
| Sucralose | 5 mg |
| Black pepper extract | 1.5 mg |
| Citric acid anhydrous | 675 mg |
| Flavouring substances | 140 mg |
| Water | 92 g |

The liquid food supplement realized as described above administered to humans increased the proliferation and motility of connective tissue cells and the synthesis of ECM proteins. In particular, the liquid food supplement intake reduced skin dryness, improved skin firmness and elasticity and exerted a putative anti-ageing effect.

## Claims

1. A food supplement comprising collagen, at least one glucosamine derivative selected between glucosamine hydrochloride and N-acetylglucosamine, L-carnitine, hyaluronic acid, a black pepper extract and a maca extract.

2. A food supplement according to claim 1, wherein said collagen is present in an amount between 1 and 10 g/50 ml, preferably 2 and 7.5 g/50 ml, more preferably 3 and 5 g/50 ml.

3. A food supplement according to claim 1 or claim 2, wherein said hyaluronic acid is present in an amount between 0.25 and 10 mg/50 ml, preferably in an amount between 0.75 and 6 mg/50 ml, more preferably in an amount between 1 and 3 mg/50 ml.

4. A food supplement according to any one of claims 1 to 3, wherein said L-carnitine is present in an amount between 1 and 400 mg/50 ml, preferably 100 and 300 mg/50 ml, more preferably 150 and 250 mg/50 ml.

5. A food supplement according to any one of claims 1 to 4, wherein said glucosamine hydrochloride is present in an amount between 1 and 3000 mg/50 ml, preferably in an amount between 500 and 1800 mg/50 ml, more preferably in an amount between 750 and 1250 mg/50 ml.

6. A food supplement according to any one of claims 1 to 5, wherein said N-acetylglucosamine is present in an amount between 1 and 30 mg/50 ml, preferably in an amount between 2 and 15 mg/50 ml, more preferably in an amount between 3 and 7 mg/50 ml.

7. A food supplement according to any one of the preceding claims, wherein said supplement is a beverage further comprising at least one among: vitamins, minerals, additives and flavouring substance.

8. A food supplement according to claim 7, wherein said vitamins are selected among: vitamin B₁, vitamin B₂, vitamin B₆, vitamin B₁₂, vitamin C, vitamin D, biotin.

9. A food supplement according to claim 7 or 8, wherein said additive is selected among: citric acid anhydrous, phosphoric acid, lactic acid, tartaric acid, DL-malic acid and sucralose.

10. A food supplement according to any one of claims 7 to 9, wherein said flavouring substance is selected among: mango essential oil, apple essential oil.

11. The use of a food supplement according to any one of the preceding claims to exert an anti-ageing effect.

## Patentansprüche

1. Nahrungsergänzungsmittel umfassend Kollagen, wenigstens ein Glucosaminderivat, ausgewählt aus Glucosaminhydrochlorid und N-Acetylglucosamin, L-Carnitin, Hyaluronsäure, einen Schwarzpfefferextrakt und einen Macaextrakt.

2. Nahrungsergänzungsmittel gemäß Anspruch 1, wobei das Kollagen in einer Menge zwischen 1 und 10 g/50 ml, vorzugsweise 2 und 7,5 g/50 ml, stärker bevorzugt 3 und 5 g/50 ml vorhanden ist.

3. Nahrungsergänzungsmittel gemäß Anspruch 1 oder Anspruch 2, wobei die Hyaluronsäure in einer Menge zwischen 0,25 und 10 mg/50 ml, vorzugsweise in einer Menge zwischen 0,75 und 6 mg/50 ml, stärker bevorzugt in einer Menge zwischen 1 und 3 mg/50 ml vorhanden ist.

4. Nahrungsergänzungsmittel gemäß einem der Ansprüche 1 bis 3, wobei das L-Carnitin in einer Menge zwischen 1 und 400 mg/50 ml, vorzugsweise 100 und 300 mg/50 ml, stärker bevorzugt 150 und 250 mg/50 ml vorhanden ist.

5. Nahrungsergänzungsmittel gemäß einem der Ansprüche 1 bis 4, wobei das Glucosaminhydrochlorid in einer Menge zwischen 1 und 3000 mg/50 ml, vorzugsweise in einer Menge zwischen 500 und 1800 mg/50 ml, stärker bevorzugt in einer Menge zwischen 750 und 1250 mg/50 ml vorhanden ist.

6. Nahrungsergänzungsmittel gemäß einem der Ansprüche 1 bis 5, wobei das N-Acetylglucosamin in einer Menge zwischen 1 und 30 mg/50 ml, vorzugsweise in einer Menge zwischen 2 und 15 mg/50 ml, stärker bevorzugt in einer Menge zwischen 3 und 7 mg/50 ml vorhanden ist.

7. Nahrungsergänzungsmittel gemäß einem der vorangehenden Ansprüche, wobei das Ergänzungsmittel ein Getränk ist, das außerdem wenigstens eines der folgenden umfasst: Vitamine, Mineralien, Zusätze und Aromastoff.

8. Nahrungsergänzungsmittel gemäß Anspruch 7, wobei die Vitamine ausgewählt sind aus: Vitamin B₁, Vitamin B₂, Vitamin B₆, Vitamin B₁₂, Vitamin C, Vitamin D, Biotin.

9. Nahrungsergänzungsmittel gemäß Anspruch 7 oder 8, wobei der Zusatz ausgewählt ist aus: wasserfreier Zitronensäure, Phosphorsäure, Milchsäure, Weinsäure, DL-Äpfelsäure und Sucralose.

10. Nahrungsergänzungsmittel gemäß einem der Ansprüche 7 bis 9, wobei der Aromastoff ausgewählt ist aus: etherischem Mangoöl, etherischem Apfelöl.

11. Verwendung eines Nahrungsergänzungsmittels gemäß einem der vorangehenden Ansprüche zum Ausüben einer Anti-Aging-Wirkung.

## Revendications

1. Complément alimentaire comprenant du collagène, au moins un dérivé de glucosamine choisi parmi l'hydrochlorure de glucosamine et la N-acétylglucosamine, la L-carnitine, l'acide hyaluronique, un extrait de poivre noir et un extrait de maca.

2. Complément alimentaire selon la revendication 1, dans lequel ledit collagène est présent dans une quantité de 1 à 10 g/50 ml, de préférence de 2 à 7,5 g/50 ml, encore mieux de 3 à 5 g/50 ml.

3. Complément alimentaire selon la revendication 1 ou la revendication 2, dans lequel ledit acide hyaluronique est présent dans une quantité de 0,25 à 10 mg/50 ml, de préférence dans une quantité de 0,75 à 6 mg/50 ml, encore mieux dans une quantité de 1 à 3 mg/50 ml.

4. Complément alimentaire selon l'une quelconque des revendications 1 à 3, dans lequel ladite L-carnitine est présente dans une quantité de 1 à 400 mg/50 ml, de préférence de 100 à 300 mg/50 ml, encore mieux de 150 à 250 mg/50 ml.

5. Complément alimentaire selon l'une quelconque des revendications 1 à 4, dans lequel ledit hydrochlorure de glucosamine est présent dans une quantité de 1 à 3 000 mg/50 ml, de préférence dans une quantité 500 à 1 800 mg/50 ml, encore mieux dans une quantité de 750 à 1 250 mg/50 ml.

6. Complément alimentaire selon l'une quelconque des revendications 1 à 5, dans lequel ladite N-acétylglucosamine est présente dans une quantité de 1 à 30 mg/50 ml, de préférence dans une quantité de 2 à 15 mg/50 ml, encore mieux dans une quantité de 3 à 7 mg/50 ml.

7. Complément alimentaire selon l'une quelconque des revendications précédentes, dans lequel ledit complément est une boisson comprenant de plus au moins un parmi : des vitamines, des minéraux, des additifs et une substance aromatisante.

8. Complément alimentaire selon la revendication 7, dans lequel lesdites vitamines sont choisies parmi : la vitamine B₁, la vitamine B₂, la vitamine B₆, la vitamine B₁₂, la vitamine C, la vitamine D, la biotine.

9. Complément alimentaire selon la revendication 7 ou 8, dans lequel ledit additif est choisi parmi : l'acide citrique anhydre, l'acide phosphorique, l'acide lactique, l'acide tartarique, l'acide DL-malique et le sucralose.

10. Complément alimentaire selon l'une quelconque des revendications 7 à 9, dans lequel ladite substance aromatisante est choisie parmi : l'huile essentielle de mangue, l'huile essentielle de pomme.

11. Utilisation d'un complément alimentaire selon l'une quelconque des revendications précédentes pour exercer un effet anti-vieillissement.
